## Europäisches Patentamt
## European Patent Office
## Office européen des brevets

(19)

(11) Veröffentlichungsnummer: **0 074 506**
**B1**

(12) **EUROPÄISCHE PATENTSCHRIFT**

(45) Veröffentlichungstag der Patentschrift:
24.10.84

(21) Anmeldenummer: 82107571.0

(22) Anmeldetag: **19.08.82**

(51) Int. Cl.³: **C 07 C  49/08,** C 07 C  45/49,
C 07 C  45/83, C 07 C  45/82,
C 07 C  45/84, C 07 C  45/80

(54) Verfahren zur Abtrennung von Aceton aus Carbonylierungsgemischen.

(30) Priorität: 11.09.81  DE 3136027

(43) Veröffentlichungstag der Anmeldung:
23.03.83 Patentblatt 83/12

(45) Bekanntmachung des Hinweises auf die Patenterteilung:
24.10.84 Patentblatt 84/43

(84) Benannte Vertragsstaaten:
AT BE CH DE FR GB IT LI NL

(56) Entgegenhaltungen:
FR - A - 2 152 768
FR - A - 2 445 309
US - A - 2 691 669

(73) Patentinhaber: HOECHST AKTIENGESELLSCHAFT,
Postfach 80 03 20, D-6230 Frankfurt am Main 80 (DE)

(72) Erfinder: Erpenbach, Heinz, Dr., Oberbuschweg 22,
D-5000 Köln 50 (DE)
Erfinder: Gehrmann, Klaus, Dr.,
Geschwister-Scholl-Strasse 32, D-5042 Erftstadt (DE)
Erfinder: Lork, Winfried,
Siegfried-von-Westerburg-Strasse 14, D-5042 Erftstadt
(DE)
Erfinder: Prinz, Peter, von Geyr-Ring 104, D-5030 Hürth
(DE)

## Beschreibung

Die Erfindung betrifft ein Verfahrer zur Abtrennung von Aceton aus Reaktionsgemischen, welche bei der Umsetzung von Methylacetat und/oder Dimethylether mit Kohlenmonoxid und ggf. Wasserstoff in Gegenwart eines aus Carbonylkomplexen von Edelmetallen der Gruppe VIII der Periodensystems, Essigsäure, einer Organophosphor- oder Organostickstoffverbindung, Methyljodid und ggf. Verbindungen carbonylbildender Nichtedelmetalle bestehenden Katalysatorsystems, z.B. gemäss DE-OS Nrn. 2450965, 2836084, 2939839 und 2941232, erhalten wurden.

Die DE-OS Nr. 2952516 (= US-PS Nr. 4252748) beschreibt ein Verfahren zur Gewinnung von Aceton aus den flüchtigen Bestandteilen des Reaktionsgemisches, das bei der Umsetzung von Methylacetat mit Kohlenmonoxid und Wasserstoff in Gegenwart eines Edelmetalls der Gruppe VIII des Periodensystems und von Methyljodid erzeugt wird, welches dadurch gekennzeichnet ist, dass man in dem Gemisch der flüchtigen Bestandteile durch Einführung von Aceton in den Carbonylierungsbereich ein Molverhältnis von Aceton zu Methyljodid von wenigstens 1:10 einstellt, dieses Gemisch aus flüchtigen Bestandteilen einer fraktionierten Destillation zur Abtrennung praktisch des gesamten Methyljodids und eines Teils des Acetons und Methylacetats als Destillat unterwirft, wobei die Menge an abgetrenntem Aceton praktisch der in die Reaktion eingeführten Menge entspricht, dass man das übrige Aceton und Methylacetat aus dem Rückstand dieser Destillation abdestilliert und danach das Aceton aus der Methylacetat/Aceton-Mischung gewinnt.

Die Isolierund des bei der Reaktion entstandenen Acetons aus dem Aceton/Methylacetat-Gemisch erfolgt über eine azeotrope Destillation mit $C_5$-Kohlenwasserstoffen gemäss US-PS Nr. 2704271, anschliessender Extraktion des Aceton/$C_5$-Kohlenwasserstoff-Gemisches mit Wasser und Fraktionierung des Acetons aus der Wasserphase. Bei dieser Betriebsweise muss also soviel Aceton mit dem im Kreislauf befindlichen Methyljodid in den Carbonylierungsbereich eingeführt werden, wie es der azeotropen Zusammensetzung des Methyljodid/Aceton-Gemisches entspricht.

Vorliegende Erfindung unterscheidet sich vom Stand der Technik u.a. vorteilhaft dadurch, dass das vorzugsweise in den Carbonylierungsreaktor zurückgeführte Methyljodid nur äusserst geringe Mengen Aceton enthält. Das Aceton übt nämlich sowohl auf die Aktivität des Katalysators als auch auf die unerwünschte Bildung organischer Folgeprodukte einen negativen Einfluss aus.

Die Abtrennung des Acetons aus dem nach der Katalysatorentfernung erhaltenen Reaktionsgemisch oder gegebenenfalls aus dessen Leichtsiedergemisch ist nicht ohne weiteres möglich. Die Leichtsiederkomponenten Methyljodid, Aceton und Methylacetat bilden azeotrope Gemische, so dass eine Reindarstellung des einzelnen Komponenten durch eine fraktionierte Destillation nicht gelingen kann. Daneben ist aber auch die destillative Trennung der reinen Komponenten von den jeweiligen azeotropen Gemischen aufgrund sehr enger Siedepunktsdifferenzen kaum oder nur mit grösstem Aufwand möglich. So liegt beispielsweise zwischen den Siedepunkten des Methyljodid/Aceton-Azeotrops mit 42,4° C und des Methyljodids mit 42,5° C nahezu keine Differenz. Ähnlich verhalten sich die Siedepunktsdifferenzen zwischen dem Aceton/Methylacetat-Azeotrop mit 55° C und Aceton mit 56,2° C bzw. Methylacetat mit 57,2° C.

Das Verfahren der Erfindung ist nun dadurch gekennzeichnet, dass man das von der Katalysatorlösung abdestillierte Reaktionsgemisch, welches Essigsäureanhydrid, Essigsäure, Ethylidendiacetat, Methyljodid, Aceton und Methylacetat enthält, als solches oder nur seinen aus Methylacetat, Methyljodid und Aceton bestehenden Leichtsiederanteil ganz oder Teilweise einer extraktiven Destillation mit Essigsäure unterzieht, dabei reines Methyljodid abdestilliert, während man aus dem Essigsäureextrakt ein Aceton/Methylacetat-Gemisch abdestilliert, dieses in bekannter Weise auftrennt und anschliessend das Extraktionsmittel Essigsäure, gegebenenfalls nach Abtrennung der bei der Reaktion gebildeten Endprodukte Essigsäureanhydrid, Ethylidendiacetat und Essigsäure, wieder in die Extraktionsstufe zurückführt.

Weiterhin kann das Verfahren der Erfindung bevorzugt und wahlweise dadurch gekennzeichnet sein, dass man

a) das von der Katalysatorlösung abdestillierte Reaktionsgemisch mit Essigsäure im Gewichtsverhältnis (0,75 - 7,5) : 1 extraktiv destilliert;

b) aus dem von der Katalysatorlösung abdestillierten Reaktionsgemisch die Leichtsieder Methylacetat, Methyljodid und Aceton gemeinsam abdestilliert und anschliessend im Gewichtsverhältnis Leichtsieder/Essigsäure von (0,5 - 5) : 1 extraktiv destilliert;

c) das bei der extraktiven Destillation abdestillierende reine Methyljodid in die Carbonylierungsreaktion zurückführt;

d) aus dem Aceton/Methylacetat-Gemisch unter Zugabe von $C_5$-Kohlenwasserstoffen ein Aceton/$C_5$-Kohlenwasserstoff-Azeotrop abdestilliert;

e) das Aceton/$C_5$-Kohlenwasserstoff-Azeotrop durch Gegenstromextraktion mit Wasser auftrennt und ggf. das Aceton aus dem Wasser abstreift;

f) das Aceton/$C_5$-Kohlenwasserstoff-Azeotrop durch extraktive Destillation mit Essigsäure in den $C_5$-Kohlenwasserstoff und ein Aceton/Essigsäure-Gemisch auftrennt, das durch fraktionierte Destillation zerlegt wird;

g) das bei der Abdestillation des Aceton/$C_5$-Kohlenwasserstoff-Azeotrops zurückbleibende reine Methylacetat in die Carbonylierungsreaktion zurückführt.

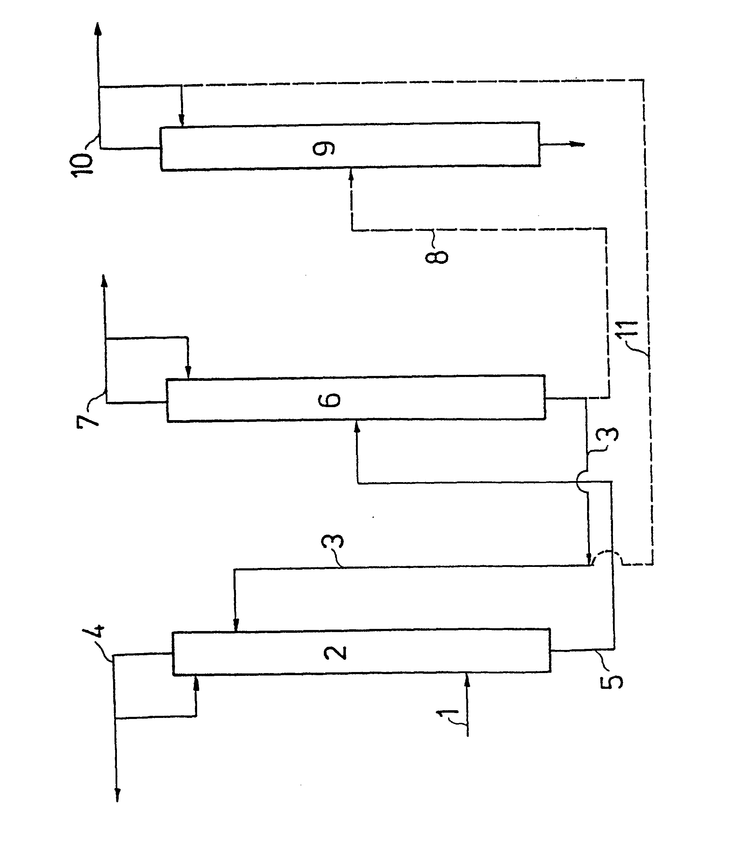

Das aus dem Carbonylierungsreaktor abfliessende Reaktionsgemisch wird zunächst von den gasförmigen Produkten wie nichtumgesetztem Kohlenmonoxid und ggf. Wasserstoff sowie Methan und Kohlendioxid befreit. In einer nachfolgenden Verdampfungsapparatur wird vom Katalysatorsystem abdestilliert. Das Destillat gelangt dann gasförmige oder vorzugsweise in verflüssigter Form direkt in die extraktive Destillationskolonne oder wird gegebenenfalls zuerst in die Leichtsieder Methylacetat, Methyljodid, Aceton als Destillat einerseits und Essigsäure, Essigsäureanhydrid und Ethylidendiacetat als Sumpfprodukt andererseits zerlegt. In diesem Falle werden die Leichtsieder der extraktiven Destillationskolonne zugeführt. Hier setzt nun das Verfahren der Erfindung ein, welches anhand des Fliessschemas der Zeichnung näher erläutert wird.

Katalysatorfreies Reaktionsgemisch oder ggf. dessen Leichtsiedergemisch gelangen über Leitung 1 in den unteren Teil der extraktiven Destillationskolonne 2 mit insgesamt 30 Böden unterhalb des Kopfes wird über Leitung 3 die zur Extraktion notwendige Menge Essigsäure zudosiert. Bezogen auf eingesetztes Reaktionsgemisch oder Leichtsiedergemisch beträgt das Gewichtsverhältnis Einsatzprodukt/Essigsäure (0,5 - 7,5): 1. Unter einem Rückfluss von 1 bis 10 wird am Kopf der extraktiven Destillationskolonne 2 bei 42 bis 43° C über Leitung 4 acetonfreies Methyljodid abgezogen und dem Carbonylierungsreaktor zugeführt. Das Sumpfprodukt der extraktiven Destillation 2 strömt über Leitung 5 in die Kolonne 6 mit 15 Böden, in der bei 56 bis 57° C Kopftemperatur und einem Rückfluss von 0,5 bis 5 das Aceton/Methylacetat-Gemisch über Leitung 7 als Destillat erhalten wird. Der Sumpfablauf der Kolonne 6 enthält bei Einsatz von Leichtsiedergemisch ausschliesslich Essigsäure, die über Leitung 3 zur Kolonne 2 zurückgelangt. Bei Einsatz von katalysatorfreiem Reaktionsgemisch in die Extraktion 2 besteht der Sumpfablauf der Kolonne 6 neben Essigsäure zusätzlich aus Essigsäureanhydrid und Ethylidendiacetat und wird über Leitung 8 der Destillationskolonne 9 mit 40 Böden zugeführt. Hier fällt unter einem Druck von 150 mbar bei 69 bis 70° C Kopftemperatur und einem Rückfluss von 0,1 bis 5 die Essigsäure über Leitung 10 als Destillat an, die nach Abzug gebildeter Essigsäure über die Leitungen 11 und 3 zur Extraktion 2 zurückgeführt wird.

Das Aceton/Methylacetat-Gemisch wird nach bekanntem Stand der Technik (nicht gezeichnet) in einer weiteren Kolonne mit Hilfe eines $C_5$-Kohlenwasserstoffgemisches durch Azeotropdestillation zerlegt. Hierbei beträgt das Gewichtsverhältnis Aceton zu $C_5$-Kohlenwasserstoff 1 : (5 - 8). Als Destillat werden das Aceton/$C_5$-Kohlenwasserstoff-Azeotrop und als Sumpfprodukt kohlenwasserstofffreies Methylacetat erhalten, welches wiederum in den Carbonylierungsreaktor gelangt. Das Aceton/$C_5$-Kohlenwasserstoffgemisch wird in bekannter Weise durch Gegenstromextraktion mit Wasser getrennt und Aceton durch Abstreifen aus dem Wasser entfernt. Als neue Alternative bietet sich eine weitere extraktive Destillation mit Essigsäure an, wobei als Destillat des $C_5$-Kohlenwasserstoff und als Sumpfprodukt ein Aceton/Essigsäure-Gemisch erhalten wird, das durch fraktionierte Destillation in seine Einzelkomponenten zerlegt werden kann.

*Beispiel 1*

Stündlich werden dem Carbonylierungsreaktor 9360 g Reaktionsprodukt entnommen. Nach Abtrennung der gasförmigen Bestandteile und der Katalysatorlösung wird ein Produktstrom von 6170 g mit etwa 0,3 Gew.-% Aceton, 16,8 Gew.-% Methyljodid, 31,2 Gew.-% Methylacetat, 15,3 Gew.-% Essigsäure, 36,2 Gew.-% Essigsäureanhydrid und 0,2 Gew.-% Ethylidendiacetat erhalten. Zur Abtrennung des Acetons wird dieser Strom auf den 10. Boden der aus insgesamt 30 Böden bestehenden Kolonne 2 eindosiert, die bei einer Sumpftemperatur von ca. 95° C, einem Druck von 1 bar und einem Rückfluss von 10 betrieben wird. Zur Extraktion des Acetons werden auf den 25. Boden 2000 g/h Essigsäure aufgegeben. Dabei stellt sich am Kopf eine Temperatur von 42° C ein. Es werden 1040 g/h Destillat mit 99,8 Gew.-% Methyljodid und 0,2 Gew.-% Methylacetat sowie 7130 g Bodenprodukt mit etwa 0,27 Gew.-% Aceton, 27 Gew.-% Methylacetat, 41,3 Gew.-% Essigsäure, 31,3 Gew.-% Essigsäureanhydrid und 0,15 Gew.-% Ethylidendiacetat erhalten. Das Destillat wird dem Carbonylierungsreaktor zugeführt. Das Sumpfprodukt der Kolonne 2 wird in Kolonne 6 mit 15 Böden bei Sumpf- und Kopftemperaturen von 130 bis 135 bzw. 57° C und einem Rückfluss von 3 zerlegt. Dabei fallen 1950 g/h Destillat mit etwa 1 Gew.-% Aceton und 99 Gew.-% Methylacetat sowie 5180 g/h Sumpfablauf mit etwa 56,7 Gew.-% Essigsäure, 43,1 Gew.-% Essigsäureanhydrid und 0,2 Gew.-% Ethylidendiacetat an.

Das Destillat wird in bekannter Weise in einer weiteren Kolonne zur Entfernung des Acetons einer azeotropen Destillation mit n-Pentan unterworfen. Bei Sumpf- und Kopftemperaturen von 58 bzw. 34° C sowie einem Rückfluss von 20 werden als Destillat 170 g/h Aceton/-n-Pentan-Gemisch erhalten, das mit etwa 11,5 Gew.-% Aceton das gesamte Aceton enthält. Als Bodenprodukt fallen 1930 g/h Methylacetat an, die dem Carbonylierungsraktor zugeleitet werden. Das Gemisch aus Aceton und n-Pentan wird in einer Extraktionskolonne mit 350 g/h Wasser extrahiert. Das am Kopf der Kolonne abfliessende n-Pentan wird der acetonhaltigen Ablauf werden in einer letzten Kolonne bei Normaldruck, einer Sumpf- und Kopftemperatur von 100 bzw. 56° C sowie einem Rückfluss von 5 als Destillat 18 g/h Aceton isoliert. Das als Bodenprodukt abfliessende Wasser wird auf die Extraktionskolonne zurückgeleitet.

Zur Entfernung der Essigsäure gelangt das Sumpfprodukt der zur Aceton/Methylacetat-Abtrennung genutzten Kolonne 6 in die unter einem Druck von 150 mbar betriebene Fraktionierkolonne 9. Bei Sumpf- und Kopftemperaturen von 97 bzw. 69° C sowie einem Rücklauf von 2 wird am Kopf der Kolonne 9 die gesamte zur Extraktion verwendete sowie die bei der Reaktion gebildete Essigsäure, die abgezogen wird, insgesamt etwa 2940 g, gewonnen.

*Beispiel 2*

Stündlich werden dem Carbonylierungsreaktor 23 450 g Reaktionsprodukt entnommen. Nach Abtrennung der gasförmigen Bestandteile, der Katalysatorlösung sowie von Essigsäure, Essigsäureanhydrid und Ethylidendiacetat wird das aus 34,3 Gew.-% Methyljodid, 64,8 Gew.-% Methylacetat und 0,8 Gew.-% Aceton bestehende Leichtsiedergemisch in einer Menge von 10 245 g/h erhalten, das nach Abzug eines Teilstromes in die Carbonylierung zurückgeführt wird. Dieser Teilstrom von 1825 g/h wird zur Extraktion von Aceton entsprechend dem Beispiel 1 dem unteren Teil der Kolonne 2 zugeführt, an deren Kopf 1000 g/h Essigsäure aufgegeben werden. Bei Sumpf- und Kopftemperaturen von 74 bzw. 42° C, einem Druck von 1 bar und einem Rückfluss von 10 werden 629 g/h Destillat mit 99,8 Gew.-% Methyljodid und 0,2 Gew.-% Methylacetat sowie 2196 g/h Bodenprodukt mit etwa 0,7 Gew.-% Aceton, 53,8 Gew.-% Methylacetat und 45,5 Gew.-% Essigsäure erhalten. Das Destillat wird zum Carbonylierungsreaktor zurückgeführt. Das aus Kolonne 2 abfliessende Bodenprodukt wird in Kolonne 6 unter Normaldruck und bei Sumpf- und Kopftemperaturen von 120 bzw. 57° C sowie einem Rückfluss von 3 in 1196 g/h Destillat mit etwa 1,25 Gew.-% Aceton und 98,75 Gew.-% Methylacetat sowie 1000 g/h Essigsäure als Sumpfprodukt zerlegt. Die Essigsäure gelangt wieder in Kolonne 2.

Das Aceton/Methylacetat-Gemisch wird zur azeotropen Entfernung des Acetons mittels Pentangemischen der Azeotropkolonne zugeleitet. Bei Sumpf- und Kopftemperaturen von 58 bzw. 34° C und einem Rückfluss von 20 werden am Kopf 115 g/h Destillat mit 13 Gew.-% Aceton und 87 Gew.-% Pentan abgezogen. Das aus dem Verdampfer abfliessende Methylacetat wird in einer Menge von 1181 g/h dem Carbonylierungsreaktor wieder zugeführt. Die Auftrennung von Aceton und Pentan aus dem azeotropen Gemisch erfolgt in einer weiteren extraktiven Destillation. Hierzu werden 400 g/h Essigsäure unterhalb des Kopfes und die 115 g/h Aceton/Pentan-Gemisch in den unteren Teil der Kolonne eindosiert. Bei Sumpf- und Kopftemperaturen von 118 bzw. 36° C und einem Rückfluss von 8 werden als Destillat das Pentangemisch sowie 415 g/h Bodenprodukt mit etwa 3,6 Gew.-% Aceton und 96,4 Gew.-% Essigsäure erhalten. Aus dem Aceton/Essigsäure-Gemisch werden dann in einer Fraktionierkolonne bei einer Sumpf- und Kopftemperatur von 120 bzw. 56° C sowie einem Rückfluss von 3 als Destillat 15 g/h Aceton gewonnen. Die aus dem Verdampfer abfliessende Essigsäure wird zur erneuten Extraktion des Aceton/Pentan-Gemisches wieder verwendet.

*Beispiel 3*

Ein aus den Carbonylierungsprodukten gemäss Beispiel 2 erhaltenes Leichtsiedergemisch wird als Teilstrom von 1151 g/h mit 2,1 Gew.-% Aceton, 86,1 Gew.-% Methyljodid und 11,8 Gew.-% Methylacetat dem unteren Teil der Kolonne 2 aufgegeben. Unterhalb des Kopfes werden zur Extraktion des Acetons 1000 g/h Essigsäure in die Kolonne eingeführt. Unter einem Rückfluss von 8, Sumpf- und Kopftemperaturen von 73 bzw. 43° C werden als Destillat 991 g/h Methyljodid abgenommen und zum Carbonylierungsreaktor zurückgeführt. Als Bodenprodukt werden 1160 g/h eines Gemisches mit etwa 2,1 Gew.-% Aceton, 11,7 Gew.-% Methylacetat und 86,2 Gew.-% Essigsäure erhalten, die zur Kolonne 6 gelangen. Hier fallen unter einem Rückfluss von 3, 120° C Sumpf- und 57° C Kopftemperatur 160 g/h Destillat mit etwa 15 Gew.-% Aceton und 85 Gew.-% Methylacetat sowie 1000 g/h Essigsäure als Bodenprodukt an.

Während die Essigsäure wieder zur Kolonne 2 zirkuliert, wird das Destillat in der Azeotropkolonne unter Zusatz von Pentan zerlegt. Bei einem Rücklauf von 20, einer Sumpf- und Kopftemperatur von 58 bzw. 34° C werden 174 g/h Destillat mit 13,8 Gew.-% Aceton und 86,2 Gew.-% Pentan sowie 136 g/h Methylacetat als Bodenprodukt erhalten. Die Auftrennung des Aceton/Pentan-Gemisches geschieht in einer Extraktionskolonne, in der das Gemisch mit 140 g/h Wasser behandelt wird. Das am Kopf ankommende Pentan strömt wieder in die Azeotropkolonne, während aus dem ablaufenden Bodenprodukt in einer weiteren Fraktionierkolonne 24 g/h Aceton unter einem Rücklauf von 3 sowie Sumpf- und Kopftemperatur von 100 bzw. 56° C isoliert werden. Das Wasser wird wieder in die Extraktion zurückgeleitet.

**Patentansprüche**

1. Verfahren zur Abtrennung von Aceton aus Reaktionsgemischen, welche bei der Umsetzung von Methylacetat und/oder Dimethylether mit Kohlenmonoxid und ggf. Wasserstoff in Gegenwart eines aus Carbonylkomplexen von Edelmetallen der Gruppe VIII des Periodensystems, Essigsäure, einer Organophosphor- oder Organostickstoffverbindung, Methyljodid und ggf. Verbindungen carbonylbildender Nichtedelmetalle bestehenden Katalysatorsystems erhalten wurden, dadurch gekennzeichnet, dass man das von der Katalysatorlösung abdestillierte Reaktionsgemisch, welches Essigsäureanhydrid, Essigsäure, Ethylidendiacetat, Methyljodid, Aceton und Methylacetat enthält, als solches oder nur seinen aus Methylacetat, Methyljodid und Aceton bestehenden Leichtsiederanteil ganz oder teilweise einer extraktiven Destillation mit Essigsäure unterzieht, dabei reines Methyljodid abdestilliert, während man aus dem Essigsäureextrakt ein Aceton/Methylacetat-Gemisch abdestilliert, dieses in bekannter Weise auftrennt und anschliessend das Extraktionsmittel Essigsäure, ggf. nach Abtrennung der bei der Reaktion gebildeten Endprodukte Essigsäureanhydrid, Ethylidendiacetat und Essigsäure, wieder in die Extraktionsstufe zurückführt.

2. Verfahren nach Anspruch 1, dadurch gekennzeichnet, dass man das von der Katalysatorlösung abdestillierte Reaktionsgemisch mit Essigsäure im Gewichtsverhältnis (0,75 - 7,5) : 1 extraktiv destilliert.

3. Verfahren nach Anspruch 1, dadurch gekennzeichnet, dass man aus dem von der Katalysatorlösung abdestillierten Reaktionsgemisch die Leichtsieder Methylacetat, Methyljodid und Aceton gemeinsam abdestilliert und anschliessend im Gewichtsverhältnis Leichtsieder/Essigsäure von (0,5 - 5) : 1 extraktiv destilliert.

4. Verfahren nach einem der vorhergehenden Ansprüche, dadurch gekennzeichnet, dass man das bei der extraktiven Destillation abdestillierte reine Methyljodid in die Carbonylierungsreaktion zurückführt.

5. Verfahren nach einem der vorhergehenden Ansprüche, dadurch gekennzeichnet, dass man aus dem Aceton/Methylacetat-Gemisch unter Zugabe von $C_5$-Kohlenwasserstoffen ein Aceton/$C_5$-Kohlenwasserstoff-Azeotrop abdestilliert.

6. Verfahren nach Anspruch 5, dadurch gekennzeichnet, dass man das Aceton/$C_5$-Kohlenwasserstoff-Azeotrop durch Gegenstromextraktion mit Wasser auftrennt und ggf. das Aceton aus dem Wasser abstreift.

7. Verfahren nach Anspruch 5, dadurch gekennzeichnet, dass man das Aceton/$C_5$-Kohlenwasserstoff-Azeotrop durch extraktive Destillation mit Essigsäure in den $C_5$-Kohlenwasserstoff und ein Aceton/Essigsäure-Gemisch auftrennt, das durch fraktionierte Destillation zerlegt wird.

8. Verfahren nach einem der Ansprüche 5 bis 7, dadurch gekennzeichnet, dass man das bei der Abdestillation des Aceton/$C_5$-Kohlenwasserstoff-Azeotrops zurückbleibende reine Methylacetat in die Carbonylierungsreaktion zurückführt.

## Claims

1. Process for separating acetone from reaction mixtures originating from the reaction of methyl acetate and/or dimethylether with carbon monoxide and optionally hydrogen, in the presence of a catalyst system consisting of carbonyl complexes of noble metals of group VIII of the periodic system of the elements, acetic acid, an organophosphorus or organonitrogen compound, methyl iodide and optionally carbonyl-yiedling compounds of common metals, wherein the reaction mixture distilled off from the catalyst solution and containing acetic anhydride, acetic acid, ethylidene diacetate, methyl iodide, acetone and methyl acetate, as such or just its low boiler fraction consisting of methyl acetate, methyl iodide and acetone, is wholly or partially subjected to an extractive distillation with acetic acid whereby pure methyl iodide is distilled off, whilst an acetone/methyl acetate mixture is distilled off from the acetic acid extract; the mixture is separated in known fashion and the acetic acid extractant is recycled into the extraction stage, optionally after separation of the final products —acetic anhydride, ethylidene diacetate and acetic acid—formed during the reaction.

2. Process as claimed in Claim 1, wherein the reaction mixture distilled off from the catalyst solution is extractively distilled with acetic acid in a ratio by weight of (0.75 - 7.5) : 1.

3. Process as claimed in Claim 1, wherein the low boilers methyl acetate, methyl iodide and acetone are distilled off jointly from the reaction mixture distilled off from the catalyst solution, and then extractively distilled in a ratio by weight of low boilers to acetic acid of (0.5 - 5) : 1.

4. Process as claimed in any of the preceding claims, wherein the pure methyl iodide distilled off during the extractive distillation is recycled into the carbonylation reaction.

5. Process as claimed in any of the preceding claims, wherein an acetone/$C_5$-hydrocarbon azeotrope is distilled off from the acetone/methyl acetate mixture with addition of $C_5$-hydrocarbons.

6. Process as claimed in Claim 5, wherein the acetone/$C_5$-hydrocarbon azeotrope is separated by countercurrent extraction with water, the acetone being optionally removed from the water by stripping.

7. Process as claimed in Claim 5, wherein the acetone/$C_5$-hydrocarbon azeotrope is separated by extractive distillation with acetic acid into the $C_5$-hydrocarbon and an acetone/acetic acid mixture, the latter being separated into its components by fractional distillation.

8. Process as claimed in any of Claims 5 to 7, wherein the pure methyl acetate retained during the distillation of the acetone/$C_5$-hydrocarbon azeotrope is recycled into the carbonylation reaction.

## Revendications

1. Procédé de séparation d'acétone de mélanges de carbonylation obtenus par réaction d'acétate de méthyle et/ou d'éther diméthylique avec le monoxyde de carbone et, éventuellement, l'hydrogène en présence d'un système catalytique constitué par des complexes carbonyles de métaux appartenant au groupe VIII de la classification périodique, de l'acide acétique, d'un composé organophosphoré ou organo-azoté, de l'iodure de méthyle et, éventuellement, des composés de métaux non nobles formant des carbonyles, caractérisé en ce que l'on soumet, en totalité ou en partie, le mélange réactionnel, séparé de la solution catalytique par distillation, qui contient de l'anhydride acétique, de l'acide acétique, du diacétate d'éthylidène, de l'iodure de méthyle, de l'acétone et de l'acétate de méthyle, tel quel ou seulement sa faction de bas points d'ébullition consistant en acétate de méthyle, iodure de méthyle et acétone, à une distillation extractive avec l'acide acétique et on chasse par distillation de l'iodure de méthyle pur tandis qu'on sépare par distillation de l'extrait d'acide acétique un mélange acétone/acétate de méthyle, on le sépare de manière connue et on renvoie l'acide acétique comme agent d'extraction dans le stade d'extraction, éventuellement après séparation des produits finals, anhydride acétique, diacétate d'éthylidène et acide acétique, formés pendant la réaction.

2 Procédé selon la revendication 1, caractérisé en ce que l'on soumet le mélange réactionnel séparé de la solution catalytique par distillation à une distillation extractive avec l'acide acétique dans un rapport pondéral de (0,75 - 7,5):1.

3. Procédé selon la revendication 1, caractérisé en ce que l'on sépare conjointement par distillation, à partir du mélange réactionnel éliminé par distillation de la solution catalytique, des composés de bas points d'ébullition, acétate de méthyle, iodure de méthyle et acétone, et on les soumet ensuite à une distillation extractive dans un rapport pondéral composés de bas points d'ébullition : acide acétique de (0,5 - 5):1.

4. Procédé selon l'une des revendications précédentes, caractérisé en ce que l'on renvoie dans la réaction de carbonylation l'iodure de méthyle pur passant dans la distillation extractive.

5. Procédé selon l'une des revendications précédentes, caractérisé en ce que l'on sépare du mélange acétone/acétate de méthyle, par distillation avec addition d'hydrocarbures en $C_5$, un azéotrope acétone/hydrocarbures en $C_5$.

6. Procédé selon la revendication 5, caractérisé en ce que l'on sépare l'azéotrope acétone/hydrocarbures en $C_5$ par extraction à contre-courant par l'eau et on sépare éventuellement l'acétone de l'eau par distillation.

7. Procédé selon la revendication 5, caractérisé en ce que l'on sépare l'azéotrope acétone/hydrocarbures en $C_5$, par distillation extractive avec l'acide acétique, en hydrocarbures en $C_5$ et en un mélange acétone/acide acétique que l'on dissocie par distillation fractionnée.

8. Procédé selon l'une des revendications 5 à 7, caractérisé en ce que l'on renvoie dans la réaction de carbonylation l'acétate de méthyle pur restant lors de la distillation de l'azéotrope acétone/hydrocarbures en $C_5$.